# EUROPEAN PATENT SPECIFICATION

(11) **EP 0 898 968 B1**
(45) Date of publication and mention of the grant of the patent: **25.06.2003**
(21) Application number: 98115722.5
(22) Date of filing: 20.08.1998
(51) Int. Cl.: A61K 38/44, C12N 11/08, A61K 47/48

(54) **Antitumor agent comprising a complex of an oxidoreductase and a polymer and a substrate of the enzyme**
Antitumormittel enthaltend einen Komplex einer Oxidoreduktase und eines Polymers und ein Substrat des Enzyms
Agent antitumoral contenant un complexe d'une oxydoréductase et d'un polymère et un substrat de l'enzyme

(30) Priority: 22.08.1997 JP 24023597
(43) Date of publication of application: 03.03.1999
(73) Proprietor: Maeda, Hiroshi, Kumamoto-shi, Kumamoto (JP)
(72) Inventor: Maeda, Hiroshi, Kumamoto-shi, Kumamoto (JP); Sawa, Tomohiro, Kumamoto-shi, Kumamoto (JP); Akaike, Takaaki, Kumamoto-shi, Kumamoto (JP)
(74) Representative: Hiltl, Elmar, Dr.

(56) References cited:
- YASUHIRO MATSUMURA AND HIROSHI MAEDA: "NEW CONCEPT FOR MACROMOLECULAR THERAPEUTICS IN CANCER CHEMOTHERAPY: MECHANISM OF TUMORITROPIC ACCUMULATION OF PROTEINS AND THE ANTITUMOR AGENT SMANCS" CANCER RESEARCH, vol. 46, no. 12, 1296, pages 6387-6392, XP002084436 USA
- J. TSUJI ET AL: "STUDIES ON ANTIGENICITY OF THE POLYETHYLENE GLYCOL (PEG)-MODIFIED URICASE" INTERNATIONAL JOURNAL OF IMMUNOPHARMACOLOGY, vol. 7, no. 5, 1985, pages 725-730, XP002084437 UNITED KINGDOM

## Description

The present invention relates to a group of antitumor agents which by themselves are not toxic and exhibit an improved tumor selective cytotoxic action.

Several antitumor agents such as mitomycin and doxorubicin have been found to exhibit their antitumor effect based on their capability of generating reactive oxygen molecular species. An earlier work by R. Bray and his co-workers (Nature, vol. 182, p. 1144-1146, 1958) and more recently T. Yoshikawa and his co-workers (Cancer Res., vol. 55, p. 1617-1620, 1995) reported that the antitumor activity of xanthine oxidase (hereinafter referred to as "XO") is achieved probably via the generation of reactive oxygen molecular species. However, a more critical reevaluation of the antitumor effect of native XO by R. Bray and J. C. Swann showed that the effect was insignificant (Structure and Bonding, vol. 11, p. 107-144, 1972, published by Elsevior, a note added in footnote of page 112). This was also confirmed again by the inventors of the present invention.

Reactive oxygen molecular species generated from those antitumor drugs exhibit an antitumor effect based on their highly cytotoxic nature. However a systemic distribution of those drugs causes undesirable side effects (J. Clin. Invest., vol. 98, p. 1253-1260, 1996). For instance, native XO leads to a binding to blood vessels after the administration into blood due to its high binding affinity to vascular endothelial cells (Biochem. J., vol. 289, 523-527, 1993). The binding of XO to blood vessels is expected to cause serious side effects such as : i) The superoxide anion radical generated from XO would oxidatively damage blood vessels; ii) The reaction between the superoxide and endogenously formed nitric oxide leads to dilatation of the blood vessels and lowers the blood pressure or thus regulates the blood pressure (Pharmacol. Rev., vol. 43, p. 109-142, 1991), which would cause hypertension due to lowered level of nitric oxide in the blood vessels (Proc. Natl. Acad. Sci. USA, vol. 88, p. 10045-10048, 1991); iii) The reaction product of superoxide and nitric oxide, namely the peroxynitrite (ONOO⁻), further oxidatively damages the blood vessels. Therefore it is not advantageous to apply native XO for clinical use. In addition, endogenous anti-XO antibody (Brit. J. Biomed. Sci., vol. 51, 124-127, 1994) may reduce the activity of XO after intravenous injection.

To enhance the drug efficacy while reducing the systemic side effects, it is necessary to deliver this antitumor enzyme selectively to the tumor tissue. The inventors of this invention previously found that macromolecular drugs and lipids preferably accumulate in the tumor tissue compared with other normal organs, and furthermore they are retained in the tumor tissue for a longer period. This phenomenon is called the EPR effect (enhanced permeability and retention effect, Cancer Res., vol. 46, p. 638-792, 1986). The enhanced therapeutic efficacy and the reduction of side effects could be achieved by increasing the molecular weight of antitumor agent (J. Controlled Rel., vol. 19, p. 315-324, 1992).

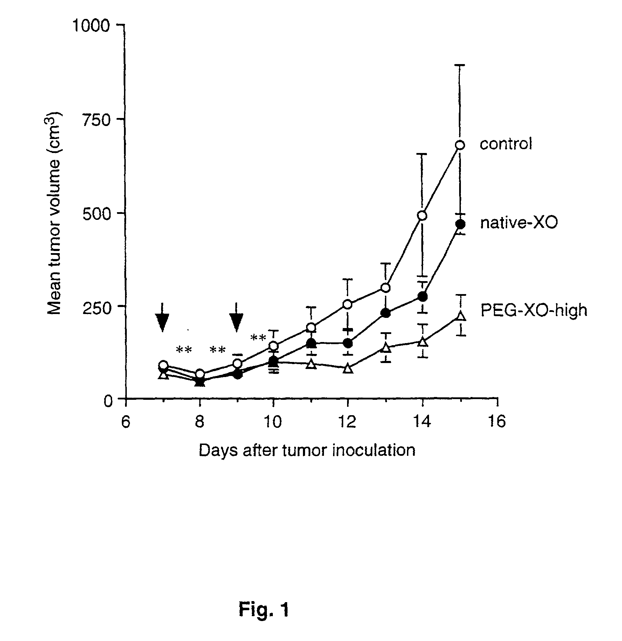

From the publication Y. Matsumura and H. Maeda, Cancer Research, Vol. 46, No. 12, 1986, pages 6387-6392, there is known "smancs" as an anticancer protein conjugated to polymers, namely a conjugate of neocarzinostatin (NCS) with a styrene-maleic acid copolymer (SMA). Besides, there are described therein some results regarding ovomucoid, bovine serum albumin (BSA), mouse serum albumin, mouse IgG and neocarzinostatin which is the parental compound of smancs (see Fig. 1, Tables 1 and 2). NCS is the antitumor material produced in the culture medium of streptomyces cartkinostaticus var. F-41 Kuroya and is not the type of antitumor agent which depends on the action of an active oxygen molecule produced from the enzyme and its substrate.

In the publication of J. Tsuji et al., International Journal of Immunopharmacology, Vol. 7, No. 5, 1985, pages 725-730, there is described the PEG-conjugated enzyme uricase, which is totally different from oxidoreductase. Uricase is used as a therapeutic agent for hyperuricemia and gout, but it is not used as an anticancer agent.

The object of the present invention is to provide a group of antitumor agents which exhibit an improved tumor selective accumulation and therefore an improved tumor selective cytotoxicity.

This object is met by the present invention according to which there are provided antitumor agents each of which is characterized by a combination of an oxidoreductase in form of xanthine oxidase, D-amino acid oxidase, glucose oxidase and/or galactose oxidase, which oxidoreductase is chemically conjugated with poly(ethylene glycol), and of a substrate of the oxidoreductase in form of hypoxanthine, xanthine, a D-amino acid, glucose and/or galactose.

The antitumor agent according to the present invention is a combination of an active enzyme component (A) in form of the above defined oxidoreductase, and of its substrate (B) in form of the above defined substrate. Upon administration of (A) and later on of (B), an active molecular species (C), such as a peroxide, is formed.

The active enzyme component(A), by its polymer conjugation, possesses a tumor targeting character. Namely, the antitumor agent exhibits a selective accumulation in tumor tissue and exerts an antitumor action if the known substrate (B) for the active enzyme component (A) is injected thereafter. Due to the enzyme reaction, active free radical components (C) (O₂ ^{·-} and H₂O₂) are formed. Both xanthine oxidase conjugated with poly(ethylene glycol) (A) and its substrate (B) show no toxicity by themselves. The potent antitumor activity is only apparent when its substrate (B) is separately administered later. By doing so, less systemic toxicity is seen while exhibiting a remarkable antitumor activity. Thus the present invention offers great benefit.

The present inventors have found a significant enhancement of the tumor accumulation of XO in tumor tissue after the XO has been chemically conjugated with poly-(ethylene glycol) (hereinafter called "PEG"; chemically conjugated XO with PEG is hereinafter called "PEG-XO"), and hence the remarkable antitumor effect of such conjugates, like PEG-XO.

Conjugation of PEG to the ε-amino group of lysine residues on the molecular surface of XO would reduce the binding affinity to endothelial cells which contain a high level of anionic charges. The masking of cationic amino groups with PEG reduces the binding of PEG-XO to endothelial cells resulting in an enhanced blood circulation time and hence in an accumulation of PEG-XO in the tumor by the EPR effect. By administrating hypoxanthine, the substrate of XO, subsequent to the administration of PEG-XO, a tumor selective antitumor action can be accomplished by the product of this enzyme reaction, which is the peroxide (Fig. 1).

As mentioned below, the above effects can be accomplished also by using other oxidoreductases besides XO with the subsequent administration of their appropriate substrates.

When using the invention in practice, a therapeutically effective amount of the oxidoreductase chemically conjugated with PEG is administered to a patient. Subsequently, a substrate of the oxidoreductase is administered additionally.

As the oxidoreductases used in the present invention, there may be cited, for example, xanthine oxidase, D-amino acid oxidase, glucose oxidase, and galactose oxidase, among which xanthine oxidase is preferably used.

If xanthine oxidase is used as the oxidoreductase, its substrate is hypoxanthine or xanthine. Substrates of D-amino acid oxidase, glucose oxidase and galactose oxidase are D-amino acids, glucose and galactose, respectively.

These oxidoreductases are chemically conjugated with PEG.

The chemical conjugation of the oxidoreductases with PEG can be carried out by conventional methods. The reaction will be carried out under relatively mild conditions, low temperature, neutral to slightly alkaline pH in an aqueous solution to avoid denaturation of the enzyme. It could be carried out also in solvents including liquid ammonia. Furthermore, a modification of amino acid residue(s) involving enzyme activity should be avoided. The conjugation of XO with PEG is described below.

XO is an oxidoreductase which takes hypoxanthine or xanthine as the substrate and produces uric acid and reactive oxygen molecular species including peroxide (O₂ ^{·-}) and H₂O₂ though for a small extent. XO can be easily obtained from bovine milk, however the source of XO is not limited to the bovine milk in the present invention.

According to the previous findings by the present inventors, PEG is a suitable polymer to be used for conjugation. It is well known that PEG is biocompatible and reduces immunogenicity of foreign proteins upon conjugation and further enhances the blood circulation time of the conjugates (cf: in Poly(ethylene glycol) Chemistry: Biotechnical and Biomedical Applications, by Harris, J.M. Ed., Plenum: New York, 1992, p. 153-169). Activated PEG can be obtained by several methods such as condensation reaction between carboxylated PEG and N-hydroxysuccinimide.

Other than ordinary single chain PEG, biantennary PEG, which has double branched PEG chains at a single conjugation point, can be also used. This biantennary PEG is synthesized using succinimidyl branched PEG. It has several advantages as a modifier of oxidoreductase, such as more reduction of immunogenicity and increased stability against temperature or various proteases.

Conjugation of XO with PEG is carried out in 50 mM sodium phosphate buffer pH 7.4, at room temperature or lower, for 30 to 60 min in this case. The extent of conjugation can be controlled by changing the feed ratio of activated PEG to lysyl residues in XO. In the present example, XO conjugated to 17-50 % of lysine residues with succinimidyl PEG was obtained by adding a 1.2-6.7 molar excess of PEG to 1 mole of lysine in XO.

PEG-XO or other biocompatible macromolecules can be selectively delivered to a solid tumor due to the EPR effect as described earlier (or see Cancer Res., vol. 46, p. 6387-6392, 1986). By administration of hypoxanthine or xanthine by injecting them intravenously after the adequate accumulation of PEG-XO in the tumor, but after clearance in the general circulation or in normal organs, XO at the tumor site generates reactive oxygen molecular species such as O₂ ^{·-} and H₂O₂, and exerts a unique antitumor action without systemic side effects.

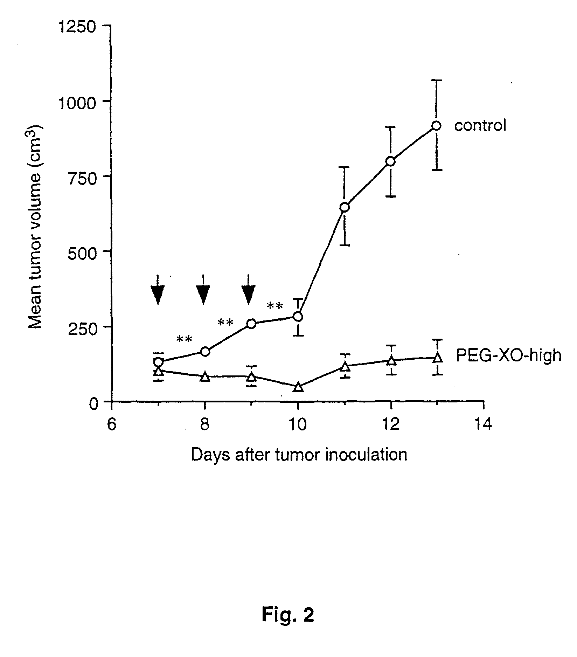

The results of this therapeutic tactics using XO and hypoxanthine against S-180 solid tumor model in mice demonstrates the significant suppression of tumor growth (Fig. 1 and 2). These results suggest that : i) reactive oxygen molecular species, which are generated by the reaction between XO and hypoxanthine, have a potent antitumor activity, and ii) the reaction between XO and hypoxanthine occurs in the solid tumor or around its periphery. In contrast thereto, native XO showed no significant antitumor activity under the conditions used (Fig. 1).

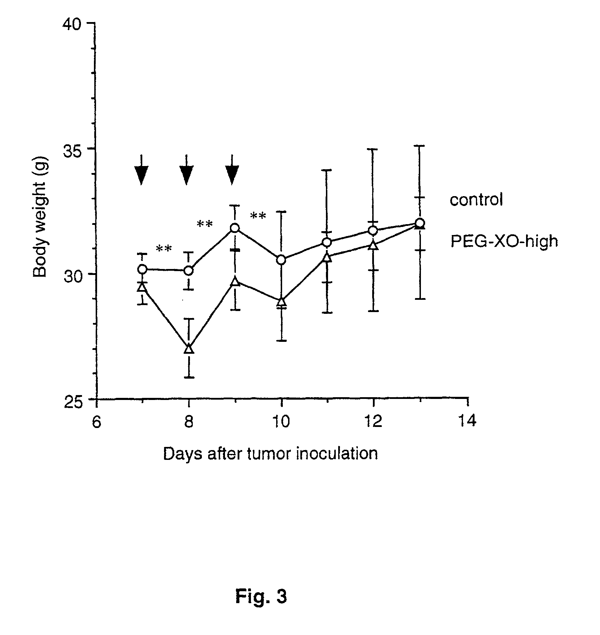

Systemic side effects of PEG-XO/hypoxanthine therapy, which were evaluated by using the body weight as a parameter, seem to be not so significant. The results showed only a transitory body weight loss, on day 8-9, but recovered on day 10 (Fig. 3). No serious or significant hematotoxicity or liver toxicity was seen.

For treating cancer by using the antitumor agents of the present invention, a therapeutically effective amount of the chemically conjugated oxidoreductase is first administrated, and after allowing a lapse of adequate time for the accumulation of the chemically conjugated oxidoreductase in the tumor, its substrate is administrated. The effective and low-toxicity dosage of the substrate is 10-100 mg/kg body weight/day.

The proper time to administrate the substrate is preferably 6 to 100 h after the administration of the chemically conjugated oxidoreductase. The antitumor agents of the present invention may be administered in the form of an injection or an oral dosage. In the case of injection, any subcutaneous, intramuscular, intravenous, intraarterial or local/direct injection is applicable. The type of preparation for oral administration may be selected optionally. Some examples of this type are tablets, granules, pills, liquid medicines, either of the oil or the aqueous type syrup, troches, and drops.

### EXAMPLES

The present invention is described by examples shown below in detail which do not limit the scope of the invention.

### Procedure of Synthesis

### Example 1: Synthesis of PEG-XO

XO from bovine milk (Sigma Chemicals, St. Louis, MO, USA) was first purified by ultrafiltration and concentrated with the use of an "Amicon system" (a molecular sieve membrane) with a PM 30 membrane (cutoff size 30, 000). The concentration of the XO solution was adjusted to 10 mg/ml protein with 50 mM sodium phosphate buffer (pH 7.4). To the XO solution, succinimide activated-PEG (Mw 5000, Shearwater Polymers, Huntsville, AL) was added at molar ratios of PEG over the ε-amino group of lysine in XO, of 1.2 and 6.7, respectively, to prepare PEG-XOs having a low and a high extent of PEG conjugation.

Unreacted PEG derivatives with functional groups, decomposed components, and other impurities were removed similarly by ultrafiltration using a PM-10 membrane as mentioned above.

The conjugates thus obtained were stored in 50 mM sodium phosphate buffer (pH 7.4) containing 1 mM sodium salicylate at 4 °C.

### Physicochemical and Biochemical Characteristics

### Example 2: Determination of the extent of the PEG conjugation

The extent of the PEG conjugation was determined by the loss of free amino groups as a result of the PEG-coupling. 2, 4, 6-Trinitrobenzenesulfonic acid was used to quantify the free amino groups of PEG-XO spectroscopically as described by Fields (Methods Enzymol., vol. 25, p. 464-468, 1972). Glycine was used as a standard amino acid. The protein concentrations of both native XO and PEG-XO were quantified by using the DC Protein Assay kit (Bio-Rad Laboratories, Hercules, CA, USA). The excess feed molar ratio of succinimidyl PEG at 1.2 or 6.7 to ε-amino groups of lysine in XO resulted in 17 % or 49 % conjugation of PEG to XO, respectively. PEG -XO having a portion of PEG of 17 % or 49 % of amino groups modified by the conjugation are hereinafter referred to as "PEG-XO -low" and "PEG-XO-high", respectively.

The molecular weight of these PEG-XO conjugates were 383 kDa or 543 kDa, respectively, which were estimated on the basis of the conjugation degree, i.e., of the loss of amino groups as obtained by the TNBS assay. The results are shown in the Table 1.

**Table 1**

| Physicochemical and Biochemical Characteristics of Native-XO and PEG-XO | | | | |
|---|---|---|---|---|
| | Feed ratio (PEG/amino group molar ratio) | % conjugated | Mw (kDa) | XO activity (U/mg protein) |
| Native-X0 | - | 0 | 298 | 2.12 |
| PEG-X0-low | 1.2 | 17 | 383 | 2.40 |
| PEG-X0-high | 6.7 | 49 | 543 | 1.15 |

### Example 3 : Size exclusion chromatography

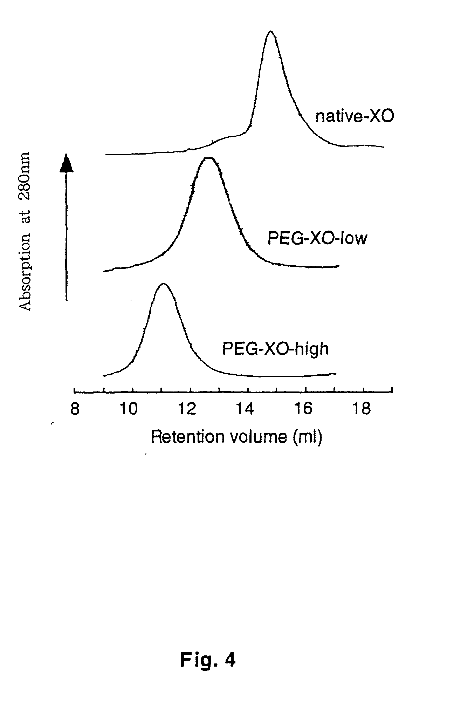

The increase of the molecular size of XO after PEG conjugation was demonstrated by means of size exclusion chromatography using the FPLC system (Pharmacia LKB, Uppsala, Sweden) equipped with a Superose 6 HR 10/30 column (Pharmacia LKB) using a mobile phase of 50 mM sodium phosphate buffer (pH 7.4). Elution of the conjugates were detected at 280 nm (Fig. 4).

### Example 4: Enzyme activity of PEG-XO

The enzyme activity was determined by quantifying the formation of uric acid from hypoxanthine by measuring the increase of absorbance at 290 nm, an absorption maximum of uric acid. The initial concentration of the substrate hypoxanthine was 50µM. The enzyme reaction was carried out in 50 mM sodium phosphate buffer (pH 7.4) at room temperature. One unit of XO activity is defined as the velocity of the formation of 1 µmol of uric acid per min. The results are shown in the Table 1.

PEG-XO-low showed slight increase of the activity (110 %) compared with Native XO. PEG-XO-high, even after a 49 % conjugation of the amino group, retained 54 % of the original enzyme activity of Native XO.

### Pharmacokinetic studies

### Example 5 : In vivo distribution of PEG-XO conjugate after intravenous injection

In vivo distribution of Native XO and PEG-XO-high was examined by using radioemitting ¹²⁵I-labeled derivatives. Both radiolabeled Native-XO and PEG-XO-high were prepared by the chloramine T method.

Sarcoma 180 tumor cells were implanted subcutaneously with 2 × 10⁶ cells in male ddY, 6-week-old mice, weighting 30-35 g, from SLC Inc., Shizuoka, Japan. The organ or tissue distribution study was performed on day 7-10 after the tumor inoculation, when the tumors were 5-7 mm in diameter, but contained no necrotic region.

¹²⁵I-Labeled Native-XO or PEG-XO-high was administered to mice via the tail vein (100 µl/mouse). After 24 h, the mice were sacrificed, and blood samples were drawn by cardiac puncture, and they were then subjected to reperfusion with heparin containing saline to remove blood components in the blood vessels of the tissues. The tumor tissue as well as normal tissues including the brain, liver, spleen, muscle, skin, heart, lung, colon, and kidney were collected and weighed. The radioactivities of those tissues were measured by a gamma counter.

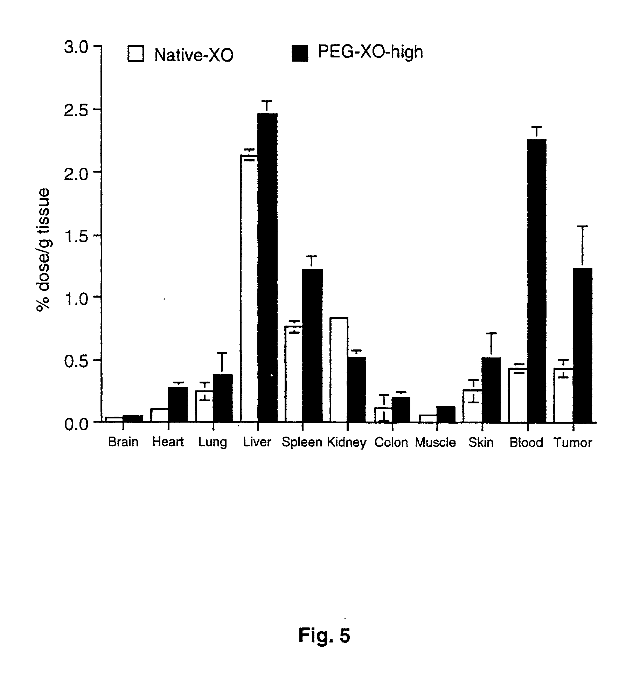

As shown in Fig. 5, PEG-XO-high was found to significantly improve both the blood and the tumor accumulation compared with that of Native-XO, whereas slight or negligible increase in accumulation in other normal organs was observed for PEG -XO-high. Furthermore, less accumulation of PEG-XO-high in the kidney was observed than with Native-XO.

### Example 6 : Time course of tumor accumulation of PEG-XO conjugate

The time course of the tumor accumulation of PEG-XO-high was examined by measuring the enzyme activity derived from PEG-XO-high in the tumor tissue. Tumor bearing mice were prepared as described above. PEG-XO-high (2U/ml, 100 µl) was injected intravenously (i.v.) to the mice. After a given period, the tumor tissue was removed as described above. The tumor tissue was then homogenized with three volumes of 20 mM potassium phosphate buffer pH 7. 6 which contained 2 mM ethylenediaminetetraacetic acid, 2 mM amidinophenylmethanesulfonyl fluoride, 10 mM dithiothreitol, 0.5 µg/ml of leupeptin. The homogenates were centrifuged at 10, 000 g for 20 min, and each supernatant was applied to a FPLC system with a Superose 6 HR 10/30 column similar to the previous section. The enzyme activity of the PEG-XO-high fraction was determined fluorometrically, i. e., the formation of fluorescent isoxanthopterin from pterin was measured with an excitation at 345 nm and an emission at 390 nm, in which hypoxanthine was replaced with pterin as substrate. The quantification was made using the calibration curve of the authentic isoxanthopterin (Aldrich Chemical, Milwaukee, WI).

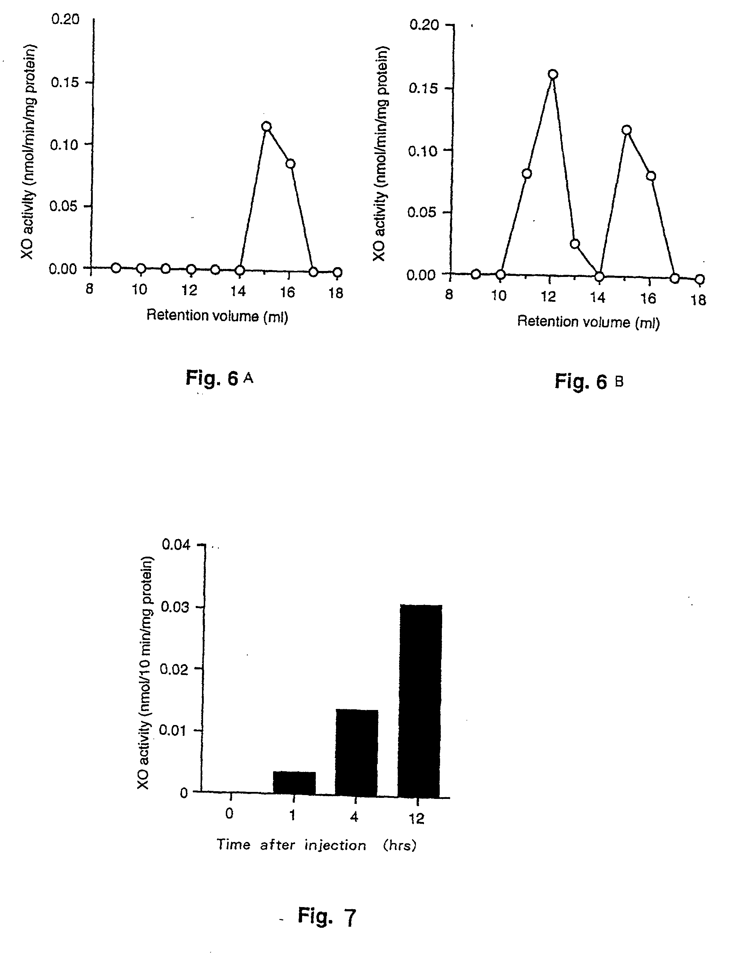

The tumor accumulation of PEG-XO-high with its enzyme activity was demonstrated by measuring the XO activity of the homogenate of the tumor before and 24 hrs after the PEG -XO high injection. The results are shown in Fig. 6.
In S-180 solid tumor tissue without the administration of PEG-XO-high, XO activity appears only in a fraction corresponding to Native XO. This means that a small amount of XO had existed in the tumor tissue endogenously (Fig 6A). On the other hand, with the solid tumor tissue after PEG-XO-high injection (0. 2 U/mouse), a new large peak of XO activity was observed at the molecular weight range different from Native XO. This new peak consisting of fraction numbers of 11, 12, and 13 corresponds to the molecular weight range of PEG-XO-high as demonstrated in Fig. 4.

Thus, Figs. 6A and 6B show that the XO activity in tumor, corresponding to the molecular weight range of PEG-XO-high, appeared after the PEG-XO-high administration.

In addition, the PEG-XO-high activity increased in a time dependent manner (Fig. 7).

### Antitumor activity in vivo

### Example 7 : Antitumor activity of PEG-XO in vivo

Sarcoma 180 were implanted subcutaneously with 2 × 10⁶ cells in male ddY, 6-week-old mice, weighting 30-35 g. When the tumors became palpable (5-7 mm in diameter), usually 7 days after implantation, the treatment was resumed.

After Native-XO or PEG-XO-high was intraveneously injected (2 times, 0.6 U/mouse, the first time 7 days and the second time 9 days after the tumor inoculation) to the mice. Hypoxanthine (13. 3mg/kg) was injected intraperitoneally six times, each time more than 6 h after the last time, as indicated by asterisks (∗) in the Fig. 1 to 3. A significant (p < 0.05) suppression of the tumor growth was observed in mice administered with PEG-XO-high. However, a similar treatment by Native XO showed no significant reduction of the tumor growth (Fig. 1). The weights of tumor 15 days after the tumor inoculation were 0.36 ±0.12 g (control), 0.31 ±0.03 g (Native XO treatment), and 0.22 ±0.05 g (PEG-XO-high treatment), respectively, which indicates that 39 % inhibition of the tumor growth was achieved by only two administrations of PEG-XO-high.

By three administrations of PEG-XO-high (on 7, 8, and 9 days after the tumor inoculation, 0.6 U/mouse) and subsequent 6 intraperitoneal injections of hypoxanthine (13. 3mg/kg) as indicated by asterisks (∗), a remarkable antitumor activity of PEG-XO-high was observed (Fig. 2). 13 days after the tumor inoculation, the weights of the tumor were 0. 57 ± 0. 24 g (control) and 0.13± 0.08 g (PEG-XO-high treatment), corresponding to a 77 % inhibition of tumor growth by PEG -XO-high.

### Example 8 : Systemic side effect of PEG-XO

In order to examine the systemic side effect of PEG-XO-high administration, the change of the body weight after the PEG -XO-high administration was investigated. PEG-XO-high was intravenously injected 3 times (on 7, 8, and 9 days after the tumor inoculation, 0.6 U/mouse). Hypoxanthine (13.3 mg/kg) was intraperitoneally injected 6 times as indicated by the asterisks (∗).

As shown in Fig. 3, a slight but significant decrease of the body weight was observed on day 8 and day 9, but then the body weight recovered to normal level on about day 10 or later. Thus, toxicity is reversible and transitory.

The invention is illustrated by the accompanying drawings.
Fig. 1 shows the effect of Native-XO and PEG-XO-high on the growth of S-180 solid tumor in ddY mice (2 times administration).
Fig. 2 shows the effect of PEG-XO-high on the growth of S-180 solid tumor in ddY mice (3 times administration).
Fig. 3 shows the body weight change of ddY mice with and without a treatment with PEG-XO-high/hypoxanthine.
Fig. 4 shows a size exclusion chromatography of Native-XO and PEG-XO.
Fig. 5 shows the body distribution of ¹²⁵I-labelled Native -XO and PEG-XO-high after intravenous injection to ddY mice bearing S-180 solid tumor.
Figs. 6A and 6B show the XO activity of S-180 solid tumor tissue before and 24 hrs after a PEG-XO high injection, respectively.
Fig. 7 shows the time dependent accumulation of PEG-XO-high in tumor tissue.

## Claims

1. Antitumor agent, **characterized by** a combination of an oxidoreductase in form of xanthine oxidase, D-amino acid oxidase, glucose oxidase and/or galactose oxidase, which oxidoreductase is chemically conjugated with poly(ethylene glycol), and of a substrate of the oxidoreductase in form of hypoxanthine, xanthine, a D-amino acid, glucose and/or galactose.

2. Antitumor agent according to claim 1, **characterized in that** the oxidoreductase is xanthine oxidase and the substrate of the oxidoreductase is hypoxanthine or xanthine.

## Patentansprüche

1. Antitumormittel, **gekennzeichnet durch** eine Kombination aus einer Oxidoreductase in Form von Xanthinoxidase, D-Aminosäureoxidase, Glucoseoxidase und/oder Galactoseoxidase, wobei die Oxidoreductase mit Poly-(ethylenglykol) chemisch konjugiert ist, und **durch** ein Substrat der Oxidoreductase in Form von Hypoxanthin, Xanthin, einer D-Aminosäure, Glucose und/oder Galactose.

2. Antitumormittel nach Anspruch 1, **dadurch gekennzeichnet, daß** die Oxidoreductase Xanthinoxidase und das Substrat der Oxidoreductase Hypoxanthin oder Xanthin ist.

## Revendications

1. Agent antitumoral, **caractérisé par** une combinaison d'une oxydoréductase sous forme de xanthine oxydase, d'acide aminé D oxydase, de glucose oxydase et/ou de galactose oxydase, laquelle oxydoréductase est chimiquement conjuguée avec le polyéthylène glycol, et d'un substrat de l'oxydoréductase sous forme d'hypoxanthine, de xanthine, d'un acide aminé D, de glucose et/ou de galactose.

2. Agent antitumoral, selon la revendication 1, **caractérisé en ce que** l'oxydoréductase est la xanthine oxydase et le substrat de l'oxydoréductase est l'hypoxanthine ou la xanthine.
